# EUROPÄISCHE PATENTSCHRIFT

(11) **EP 2 558 049 B1**
(45) Veröffentlichungstag und Bekanntmachung des Hinweises auf die Patenterteilung: **05.03.2014**
(21) Anmeldenummer: 11711517.0
(22) Anmeldetag: 25.03.2011
(51) Int. Cl.: A61F 13/476

(54) **DAMEN- ODER INKONTINENZBINDE**
SANITARY NAPKIN OR INCONTINENCE GUARD
SERVIETTE HYGIENIQUE OU PROTECTION CONTRE L'INCONTINENCE

(30) Priorität: 16.04.2010 DE 102010015559
(43) Veröffentlichungstag der Anmeldung: 20.02.2013
(73) Patentinhaber: Paul Hartmann Aktiengesellschaft, 89522 Heidenheim (DE)
(72) Erfinder: EILERS, Jörg, 89073 Ulm (DE); KESSELMEIER, Ruediger, 89542 Herbrechtingen (DE); BOEHMLER, Andreas, 89518 Heidenheim (DE); SCHWARZ, Karina, 89358 Kammeltal-Ried (DE); MANNS, Thurid, 89518 Heidenheim (DE)
(74) Vertreter: DREISS Patentanwälte PartG mbB
(86) Internationale Anmeldenummer: PCT/EP2011/054601
(87) Internationale Veröffentlichungsnummer: WO 2011/128198

(56) Entgegenhaltungen:
- WO-A1-96/38110
- US-A1- 2009 088 716

## Beschreibung

Die Erfindung betrifft eine Damen- oder Inkontinenzbinde mit um den Steg eines Unterbekleidungsstücks herum faltbaren Flügeln, wobei die Binde eine Längsrichtung und eine Querrichtung sowie einen in der Längsrichtung vorderen Endbereich, der wenigstens ein Viertel der Gesamtlängserstreckung der Binde erfasst, und einen in der Längsrichtung hinteren Endbereich, der höchstens ein Viertel der Gesamtlängserstreckung der Binde erfasst, aufweist, mit einem mit seiner längeren Abmessung in der Längsrichtung erstreckten und einen Absorptionskörper für Körperflüssigkeiten aufnehmenden Bindenhauptteil und mit zwei Flügeln auf jeder Längsseite des Bindenhauptteils, die ausgehend von dem Bindenhauptteil in Querrichtung vorstehen, so dass sie ein vorderes und ein hinteres Flügelpaar bilden, wobei das vordere Flügelpaar in Längsrichtung außerhalb des vorderen Endbereichs angeordnet ist, so dass der vordere Endbereich nicht von Flügeln flankiert ist, wobei die Flügel des vorderen und des hinteren Flügelpaars jeweils von einer vorderseitigen und von einer rückseitigen Flanke begrenzt werden.

Damen- oder Inkontinenzbinden mit Flügeln sind mehrfach beschrieben, insbesondere in WO 96/38110 A1, EP 0 731 681 B1, DE 2000 96 10 U1, WO 98/06367 A1, EP 0 511 905 B1. Ausgehend von der gattungsbildenden Damen- oder Inkontinenzbinde gemäß WO 96/38110 A1 liegt der vorliegenden Erfindung die Aufgabe zugrunde, die Handhabbarkeit des Hygieneartikels beim Anbringen an einem Unterbekleidungsstück zu verbessern und auch das Trageverhalten des Hygieneartikels weiter zu optimieren. Dabei bestehen Zielkonflikte in mehrerer Hinsicht. Einerseits ist man versucht, über eine möglichst große flächenhafte Erstreckung und Ausbildung der Flügel eine sichere Anbringung der Binde am Schrittsteg eines Unterbekleidungsstücks zu erreichen. Aufgrund der im Gebrauch dreidimensional gekrümmten Gestalt der Binde wurde mit der vorliegenden Erfindung aber erkannt, dass eine ausladende Flügelausbildung auch Nachteile mit sich bringt, indem sie die Flügel in nachteiliger Weise versteift, was sich sowohl beim Anordnen der Binde im Unterbekleidungsstück als auch im Tragegebrauch der Binde als nachteilig und unbequem herausstellt. Dies wurde mit der vorliegenden Erfindung auch dann festgestellt, wenn das hintere Flügelpaar nicht um Seitenränder der Beinöffnungen des Unterbekleidungsstücks auf dessen Außenseite herumgefaltet ist, sondern flächenhaft von innen gegen das Unterbekleidungsstück, vorzugsweise haftend fixiert, anliegt. Auch in diesem Fall wirkt sich die dreidimensionale Verformung des Hygieneartikels im Gebrauch nachteilig auf den Tragkomfort des Hygieneartikels aus.

Die Anmelderin hat in bereits erfinderischer Weise diesen Zielkonflikt erkannt und schlägt zur Vermeidung der vorausgehend geschilderten Probleme vor, eine Damen- oder Inkontinenzbinde der gattungsgemäßen Art so auszubilden, dass wenigstens 90 % der Aufsichtsfläche der Flügel des hinteren Flügelpaars innerhalb des hinteren Endbereichs angeordnet ist. Als Aufsichtsfläche der Flügel wird derjenige Flächenbereich der Flügel verstanden, der in Querrichtung außerhalb einer in Längsrichtung verlaufenden Tangente an die schmalste Stelle der Binde zwischen dem vorderen und dem hinteren Flügelpaar im eben ausgebreiteten Zustand der Binde angeordnet ist. Für die Zwecke der Bestimmung dieser Flügelfläche wird beidseits eine zur zentralen Längsmittelachse (Längsrichtung) der Damen- oder Inkontinenzbinde parallele Gerade angelegt, wobei als Berührungspunkt die schmalste Stelle der Binde zwischen vorderem und hinterem Flügelpaar gewählt wird.

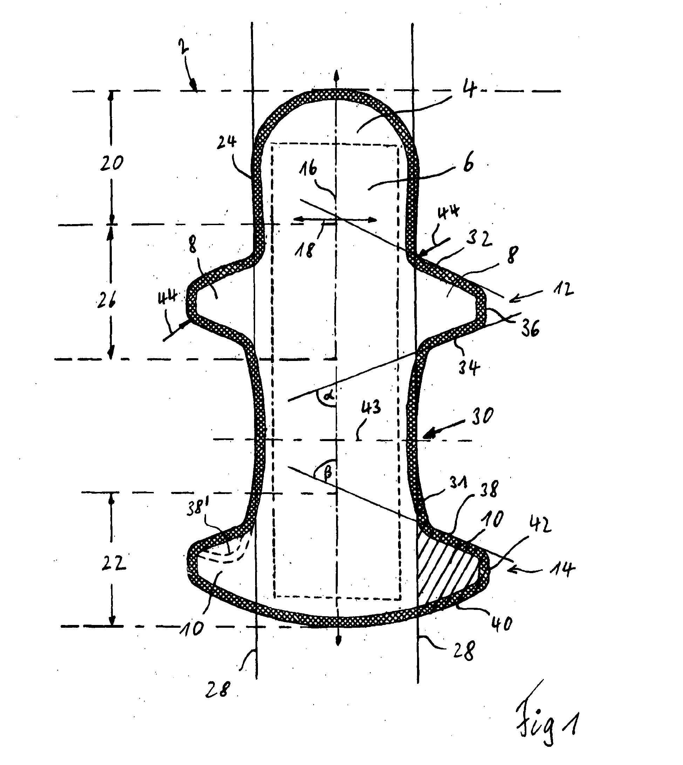

Dadurch, dass die Flügel des hinteren Flügelpaars mit ihrer flächenhaften Erstreckung im Wesentlichen vollständig in dem hinteren Endbereich der Binde vorgesehen sind, ist der vorstehend geschilderte versteifende Widerstand bei dreidimensionaler Krümmung, der auf das hintere Flügelpaar zurückzuführen ist, geringer, weil die hinteren Flügel auf einen in Längsrichtung kürzeren Bereich der Binde beschränkt angeordnet sind. Bei der erfindungsgemäßen Damen- oder Inkontinenzbinde sind die hinteren Flügel somit auch nicht direkt im Schrittbereich vorgesehen, was zu weniger Material im Schrittbereich führt. Insofern erweist es sich in weiterer Ausbildung der Erfindung als vorteilhaft, wenn der hintere Endbereich höchstens ein Fünftel (1/5), insbesondere höchstens ein Sechstel (1/6) der Gesamtlängserstreckung der Binde erfasst (vordere Randkante bis hintere Randkante in Längsrichtung 16, siehe Figur 1). Der vordere Endbereich der Binde erfasst vorzugsweise höchstens ein Drittel (1/3), insbesondere höchstens zwei Siebtel (2/7) der Gesamtlängserstreckung der Binde (vordere Randkante bis hintere Randkante in Längsrichtung 16).

Weiter erweist es sich als vorteilhaft, wenn auch die Flügel des vorderen Flügelpaars über einen in Längsrichtung begrenzten Längsabschnittsbereich der Binde angeordnet bzw. erstreckt sind. Bevorzugtermaßen sind die Flügel des vorderen Flügelpaars mit wenigstens 90 % ihrer Aufsichtsfläche innerhalb eines Längsabschnittsbereichs der Binde von höchstens ein Viertel (1/4) ihrer Gesamtlängserstreckung angeordnet. In Weiterbildung dieses Gedankens erweist es sich als vorteilhaft, wenn der genannte Längsabschnittsbereich an den vorderen Endbereich anschließt, wobei der vordere Endbereich in Längsrichtung höchstens ein Drittel (1/3), insbesondere höchstens zwei Siebtel (2/7) der Gesamtlängserstreckung der Binde erfasst. Insbesondere kann der genannte Längsabschnittsbereich das zweite Viertel der Gesamtlängserstreckung der Binde (von vorn betrachtet) erfassen. Ein flügelfreier Längsabschnittsbereich zwischen dem hinteren Endbereich des hinteren Flügelpaars und dem Längsabschnittsbereich des vorderen Flügelpaars weist eine bevorzugte Längserstreckung von wenigstens zwei Zwölftel (2/12), vorzugsweise von wenigstens ein Viertel (1/4) der Gesamtlängserstreckung der Binde auf.

Weiter erweist es sich als vorteilhaft, wenn die Erstreckung der Flügel des vorderen und/oder hinteren Flügelpaars in Querrichtung über die schmalste Stelle der Binde zwischen dem vorderen und dem hinteren Flügelpaar hinaus 20 - 70 mm, insbesondere 20 - 50 mm, insbesondere 25 - 45 mm beträgt.

Weiter erweist es sich als vorteilhaft, wenn die Aufsichtsfläche jedes Flügels des hinteren Flügelpaars jeweils einen Anteil von 3 - 7 %, weiter vorzugsweise von 4 - 6 % bezogen auf die Gesamtfläche der Damen - oder Inkontinenzbinde einnimmt. Der im Vergleich zur Gesamtfläche geringe flächenhafte Anteil des hinteren Flügels ermöglicht eine insbesondere zur Anbindung des Flügels auf der Innenseite des Unterbekleidungsstückes ausreichende Erstreckung unter Vermeidung von versteifenden Widerständen durch Materialanhäufung in Längsrichtung der Binde.

Insbesondere erweist es sich als vorteilhaft, wenn die Aufsichtsfläche jedes Flügels des vorderen Flügelpaars jeweils einen Anteil von 3 - 7 %, weiter vorzugsweise von 4 - 6 % bezogen auf die Gesamtfläche der Damen - oder Inkontinenzbinde einnimmt.

Weiter erweist es sich als vorteilhaft, wenn die Aufsichtsfläche jedes Flügels des vorderen Flügelpaars jeweils 10 - 17 cm², weiter vorzugsweise 11 - 16 cm², weiter vorzugsweise 12 - 15 cm², weiter vorzugsweise 13 - 14 cm² und/oder die jedes Flügels des hinteren Flügelpaars jeweils 10 - 17 cm², weiter vorzugsweise 11 - 16 cm², weiter vorzugsweise 12 - 15 cm², weiter vorzugsweise 13 - 14 cm² beträgt.

Die Gesamtlängserstreckung der Damen- oder Inkontinenzbinde entlang der Längsrichtung beträgt bei einer bevorzugten Ausführungsform 150 bis 400 mm, insbesondere 200 bis 350 mm, insbesondere 250 bis 300 mm, insbesondere 275 bis 290 mm.

Um eine möglichst kompakte Ausbildung und Anordnung der Flügel in einem begrenzten eher kurzen Längsabschnittsbereich der Binde zu realisieren, jedoch dennoch eine möglichst große Fläche der Flügel zur Anbindung an das Unterbekleidungsstück zu haben, erweist es sich als vorteilhaft, wenn die vorderseitigen und die rückseitigen Flanken der Flügel des vorderen und/oder hinteren Flügelpaars mit dem überwiegenden Teil ihrer Bahn in einem Winkel (α, β) > 50°, insbesondere > 60°, insbesondere > 65° zur Längsrichtung verlaufen, wobei der Winkel weiter insbesondere 60 - 85°, insbesondere 60 - 75° beträgt. Vorliegend ist dabei der kleinere der beiden Schnittwinkel zwischen der Flankenrichtung und der Längsrichtung gemeint. Bei einer derartigen Ausbildung werden sich die Flügel in Querrichtung nach außen verjüngen, wobei ihr Verlauf durchaus steil ist, was große Flügelflächen bei vergleichsweise moderater Flügelerstreckung in Längsrichtung gestattet.

Bei einer weiteren in Figur 1 hinten links nur angedeuteten Ausführungsform können die vorderseitigen Flanken der Flügel des hinteren Flügelpaars in einem Winkel > 80°, insbesondere > 90°, insbesondere 90 - 110°, insbesondere 90 - 100° zur Längsrichtung verlaufen. Danach kann bei den Flügeln des hinteren Flügelpaars die Länge der Anbindung des Flügels an den Bindenhauptteil extrem verkürzt werden, was zwar deren Faltbarkeit erleichtert, jedoch wiederum zu einer Reduzierung der Aufsichtsfläche der Flügel beiträgt, was insbesondere wiederum durch eine größere Quererstreckung ausgeglichen werden kann.

Im Hinblick auf die Konturierung der Flügel könnten beispielsweise die jeweiligen vorderseitigen Flanken verrundet unmittelbar in die rückseitigen Flanken übergehen. Es wäre aber auch denkbar, dass zwischen der vorderseitigen und der rückseitigen Flanke eines jeweiligen Flügels ein gegenüber diesen Flanken kürzerer Flankenabschnitt vorgesehen ist, der insbesondere im Wesentlichen in Längsrichtung verläuft und in den die Flanken verrundet übergehen.

Zwar wäre es denkbar, dass im Übergang zwischen einem Längsrand des Bindenhauptteils und einer vorderseitigen oder rückseitigen Flanke der Flügel ein unstetiger Konturverlauf vorgesehen ist, wodurch im Übrigen die Ausbildung einer Biege- oder Falzlinie vorgegeben werden kann. Indessen erweist es sich als vorteilhaft, wenn die Außenkontur der Binde verrundet ausgebildet ist. In Weiterführung dieses Gedankens erweist es sich als vorteilhaft, wenn ein Krümmungsradius im Übergang zwischen einem im Wesentlichen in der Längsrichtung erstreckten Längsrand des Bindenhauptteils und der vorderseitigen Flanke und/oder zwischen der vorderseitigen Flanke und dem mittleren kürzeren Flankenabschnitt und/oder zwischen dem mittleren kürzeren Flankenabschnitt und der rückseitigen Flanke und/oder zwischen der rückseitigen Flanke und dem im Wesentlichen in der Längsrichtung erstreckten Längsrand des Bindenhauptteils eines jeweiligen Flügels 5 - 12 mm, insbesondere 5 - 10 mm, insbesondere 6 - 9 mm beträgt.

Nach einem weiteren Erfindungsgedanken, der sich auch in optischer Hinsicht vorteilhaft auswirken kann, wird vorgeschlagen, die Flügelkonturen so auszubilden, dass die rückseitigen Flanken des vorderen Flügelpaars und die vorderseitigen Flanken des hinteren Flügelpaars mit dem überwiegenden Teil ihrer Bahn gerade verlaufen. Weiter kann es sich als vorteilhaft erweisen, wenn die vorderseitigen Flanken des vorderen Flügelpaars und/oder die rückseitigen Flanken des hinteren Flügelpaars bogenförmig gekrümmt, vorzugsweise unter Ausbildung einer nach außen gewölbten Kontur verlaufen, wobei solchenfalls vorzugsweise nur eine sehr leichte bogenförmige Krümmung mit einem Krümmungsradius in der Größenordnung von 100 mm und mehr, insbesondere mit einem Krümmungsradius von 120 - 180 mm, weiter insbesondere von 140 - 160 mm, weiter insbesondere von 150 - 155 mm vorgesehen ist.

Vorteilhafterweise verlaufen die vorderseitigen Flanken des vorderen Flügelpaares und die rückseitigen Flanken des hinteren Flügelpaars gekrümmt.

Die Krümmung der vorderseitigen Flanken des vorderen Flügelpaares und/oder der rückseitigen Flanken des hinteren Flügelpaars trägt in vorteilhafter Weise zur ergonomischen Passform der Inkontinenz- oder Damenbinde im Gebrauchszustand bei. Im Gebrauchszustand der Faltung der Flügel des vorderen Flügelpaares um den Schrittsteg herum auf die Außenseite des Unterbekleidungsstücks und der Anbindung der Flügel des hinteren Flügelpaares gegen die Innenseite des Unterbekleidungsstücks unterstützt die Krümmung der Flanken der Flügel die flexible Anpassung an die dreidimensionale Gestalt der Binde.

Obschon die vorderseitigen Flanken des vorderen Flügelpaars und/oder die rückseitigen Flanken des hinteren Flügelpaars nicht gerade, sondern vorzugsweise leicht bogenförmig gekrümmt verlaufen, so lässt sich hier ebenfalls eine Tangente an den Konturverlauf anlegen, die mit der Längsrichtung einen Winkel > 50°, insbesondere > 60°, insbesondere > 65° zur Längsrichtung einschließt, wobei der Winkel weiter insbesondere 60 - 85°, insbesondere 60 - 75° beträgt.

Bei einer besonders vorteilhaften Ausführungsform der Damen- oder Inkontinenzbinde sind die vorderseitigen Flanken des vorderen Flügelpaars und die rückseitigen Flanken des hinteren Flügelpaars einander entsprechend konturiert, so dass sie bei Faltung der Binde um eine in Querrichtung (also senkrecht zur Längsachse) verlaufende Achse in Deckung aufeinander bringbar sind. In Weiterführung dieses Erfindungsgedankens erweist es sich als vorteilhaft, wenn die vorderseitigen Flanken des vorderen Flügelpaars und die rückseitigen Flanken des hinteren Flügelpaars jeweils einer Kreisbahn folgen oder einer Ellipse oder Ovalform folgen, deren größere Hauptachse mit der vorerwähnten in Querrichtung verlaufenden Achse zusammenfällt.

Es erweist sich weiter als vorteilhaft, wenn die Flügel des hinteren Flügelpaars zugleich den hinteren Abschluss der Damen- oder Inkontinenzbinde bilden. Hierbei erweist es sich als vorteilhaft, wenn die rückseitigen Flanken des hinteren Flügelpaars in ergonomischer Fortsetzung ihrer Bahn nach innen aufeinander zustreben und so den hinteren Endbereich des Bindenhauptteils begrenzen. Die Flügel des hinteren Flügelpaars gehen also in gewisser Weise ergonomisch einstückig in den Bindenhauptteil über, so dass der Bindenhauptteil in Längsrichtung keinen weitergehenden Fortsatz in rückwärtiger Richtung der Binde aufweist.

Die Flügel der Damen- oder Inkontinenzbinde weisen zur Festlegung an das Unterbekleidungsstücks vorteilhafterweise Haftzonen auf. Die Haftzonen sind dabei auf der Unterseite der Flügel vorgesehen. Es erweist sich weiter als vorteilhaft, wenn die Fläche der jeweiligen Haftzone pro Flügel an die Aufsichtsfläche des Flügels angepasst ist. Die Haftzone weist je Flügel vorzugsweise eine Fläche von 20 - 50%, weiter vorzugsweise von 20 - 40% bezogen auf die Aufsichtsfläche des Flügels auf. Dabei ist die Haftzone vorteilhafterweise mittig zwischen der vorderseitigen und der rückseitigen Flanke des jeweiligen Flügels angeordnet.

Auch bei der Befestigung der Binde mittels der Haftzonen ist die insbesondere bei den Flügeln des hinteren Flügelpaares kurze Erstreckung in Längsrichtung vorteilhaft, da an sich große flächenhafte Erstreckungen der Flügel zur Vermeidung von Materialverfaltungen beim Gebrauch einen größeren Anteil an Haftzonen bedürfen, was sich wiederum nachteilig in einem versteifenden Widerstand in diesen Bereichen zeigen würde.

Weiter vorteilhaft ist der Bindenhauptteil der Damen- oder Inkontinenzbinde auf der Unterseite, also auf der dem im Gebrauch vorhandenen Unterbekleidungsstück zugewandten Seite mit weiteren Haftzonen versehen. Diese weiteren Haftzonen können an sich beliebig, insbesondere in Form von mehreren in Längsrichtung des Bindenhauptteils erstreckten Streifen angeordnet sein.

Herstellerseitig können die Damen- oder Inkontinenzbinden in eben ausgebreitetem Zustand, insbesondere in Stapelform, an den Letztverbraucher abgegeben werden. Es wäre indessen auch denkbar, dass die Binde um eine oder mehrere in Querrichtung verlaufende Faltachsen auf sich selbst gefaltet ist, so dass ihre Längserstreckung vor dem Gebrauch hierdurch erheblich reduziert ist. Unabhängig von einer solchen Faltung der Binde um eine oder mehrere Querachsen können die vorderen und/oder die hinteren Flügel auf die Oberseite oder die Unterseite des Bindenhauptteils herstellerseitig eingefaltet sein, um die Quererstreckung des Produkts bei der Lagerung, im Handel und bei der Abgabe an den Endverbraucher zu reduzieren. Eine hierfür vorgesehene Längsfaltlinie verläuft dabei typischer Weise in Längsrichtung der Binde und kann auch Randbereiche des Bindenhauptteils außerhalb der eigentlichen Flügel erfassen. Jedoch verläuft eine solche herstellerseitig vorgenommene Längsfaltlinie außerhalb des Absorptionskörpers der Binde. Es kann sich als vorteilhaft erweisen, wenn die Flügel des vorderen und/oder des hinteren Flügelpaars herstellerseitig auf eine körperzugewandte Oberseite der Binde geschlagen sind und möglicherweise einander dabei überlappen und wenn eine dann nach oben gewandte Haftbeschichtung bei den jeweiligen Flügeln je Flügelpaar von einem einzigen ablösbaren flächenhaften Schutzelement, insbesondere auf Papier- oder Folienbasis, insbesondere mit Antihaftmitteln beschichtet, überfangen ist, welches beide Flügel erfasst.

Die Flügel des vorderen und hinteren Flügelpaares umfassen vorzugsweise oder bestehen vorzugsweise aus einem die Oberseite der Damen- oder Inkontinenzbinde bildenden Deckblatt-Material und aus einem die Unterseite der Damen- oder Inkontinenzbinde bildenden Backsheet-Material. Die Flügel sind vorzugsweise frei von zwischen Deckblatt- und Backsheet-Material angeordneten Absorptionsmaterialien.

Der Absorptionskörper erstreckt sich vorzugsweise innerhalb des Bindenhauptteils, und ist insbesondere rechteckigförmig ausgebildet und in Längsrichtung der Binde eingebracht. Es ist auch denkbar, dass die Form des Absorptionskörpers in den Endbereichen dem Konturverlauf der Endbereiche des Bindenhauptteils angepasst ist.

Für sämtliche vorausgehend beschriebenen Merkmale wird Schutz in jeder beliebigen Kombination in Anspruch genommen. Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Damen- oder Inkontinenzbinde. In der Zeichnung zeigt:
- Figur 1: eine Damen- oder Inkontinenzbinde im eben ausgefalteten Zustand;
- Figur 2: die Damen- oder Inkontinenzbinde nach Figur 1 mit angedeuteter herstellerseitig vorgenommener Einfaltung der Flügel;
- Figur 3: die Damen- oder Inkontinenzbinde nach Figur 1 im eben ausgefalteten Zustand mit schematisch angedeutetem Verlauf der vorderseitigen Flanken des vorderen Flügelpaars und der rückseitigen Flanken des hinteren Flügelpaars und
- Figur 4: eine schematisch perspektivische Ansicht der Damen- oder Inkontinenzbinde nach Figur 1 im Gebrauchszustand in ein Unterbekleidungsstück eingebracht.

Figur 1 zeigt eine insgesamt mit dem Bezugszeichen 2 bezeichnete Damen- oder Inkontinenzbinde mit einem Bindenhauptteil 4, der einen im beispielhaft dargestellten Fall rechteckförmigen Absorptionskörper 6 umfasst, und mit zwei Flügeln 8 und 10 auf jeder Seite. Die vorderen Flügel 8 bilden ein vorderes Flügelpaar 12 und die hinteren Flügel 10 bilden ein hinteres Flügelpaar 14.

Mit Bezugszeichen 16 ist eine Längsrichtung und mit Bezugszeichen 18 eine hierzu senkrechte Querrichtung der Damen- oder Inkontinenzbinde 2 bezeichnet. Ferner ist in Figur 1 angedeutet ein vorderer Endbereich 20 und ein hinterer Endbereich 22. Die Längserstreckung des vorderen Endbereichs 20 erfasst wenigstens 1/4 der Gesamtlängserstreckung der Damen- oder Inkontinenzbinde, die im beispielhaft dargestellten Fall zwischen 275 - 290 mm beträgt. In diesem vorderen Endbereich 20 sind keine Flügel vorhanden, sondern der Bindenhauptteil 4 begrenzt die Binde mit seinen im Wesentlichen in Längsrichtung verlaufenden Längsrändern 24. An diesen vorderen Endbereich 20 schließt sich ein Längsabschnittsbereich 26 an, der beispielhaft höchstens 1/4 der Gesamtlängserstreckung der Damen- oder Inkontinenzbinde umfasst, und innerhalb dessen die Flügel 8 des vorderen Flügelpaars 12 mit ihrer im Wesentlichen gesamten flächenhaften Erstreckung angeordnet sind. In dem hinteren Endbereich 22 sind die Flügel 10 des hinteren Flügelpaars 14 angeordnet; die Längserstreckung des hinteren Endbereichs 22 beträgt höchstens 1/4, insbesondere höchstens 1/5 der Gesamtlängserstreckung der Damen- oder Inkontinenzbinde 2.

Zur Abgrenzung der Flügel 8, 10 vom Bindenhauptteil 4 ist in Figur 1 jeweils beidseitig eine parallele Gerade oder Tangente 28 an die schmalste Stelle 30 der Binde zwischen dem vorderen und dem hinteren Flügelpaar 12, 14 gelegt. Diejenigen Bereiche der Binde in Querrichtung 18 außerhalb dieser Geraden oder Tangente 28, einschließlich eines im beispielhaft dargestellten Fall vorgesehenen einige Millimeter breiten Siegelrands 31 der Binde werden den Flügeln 8, 10 zugeordnet. Auch sie tragen zu der gesamten Fläche oder Aufsichtsfläche der Flügel 8, 10 im Sinne der vorliegenden Erfindung bei, wobei die schmalste Stelle 30 im Zweifel die vorderen und hinteren Flügel 8, 10 voneinander trennt.

Die Flügel 8 des vorderseitigen Flügelpaars 12 werden von vorderseitigen Flanken 32 und rückseitigen Flanken 34 sowie von einem zwischen der vorderseitigen und der rückseitigen Flanke zu liegen kommenden kürzeren Flankenabschnitt 36, der im Wesentlichen in Längsrichtung 16 verläuft, begrenzt. Auch die Flügel 10 des hinteren Flügelpaars 14 werden von einer vorderseitigen Flanke 38 und einer rückseitigen Flanke 40 sowie einem mittleren kürzeren Flankabschnitt 42 begrenzt.

Die einander zugewandten Flanken 34, 38 der vorderen Flügel 8 bzw. hinteren Flügel 10 verlaufen im Wesentlichen gerade und schneiden die Längsrichtung 16 in einem Winkel α bzw. ß, der vorzugsweise > 65° beträgt und insbesondere zwischen 60 und 85° liegt. Obschon die einander abgewandten Flanken 32 und 40 der vorderen Flügel 8 bzw. der hinteren Flügel 10 nicht gerade, sondern leicht bogenförmig gekrümmt verlaufen, so lässt sich hier ebenfalls eine Tangente an den Konturverlauf anlegen (vorn dargestellt), die mit der Längsrichtung einen Winkel in dem entsprechenden Bereich 60 bis 85° einschließt. Auf diese Weise werden in der Längsrichtung 16 verhältnismäßig kurz erstreckte Flügel 8, 10 gebildet, die eine sehr gute Handhabbarkeit beim Befestigen an einem Unterbekleidungsstück aufweisen und auch einen hohen Tragkomfort vermitteln.

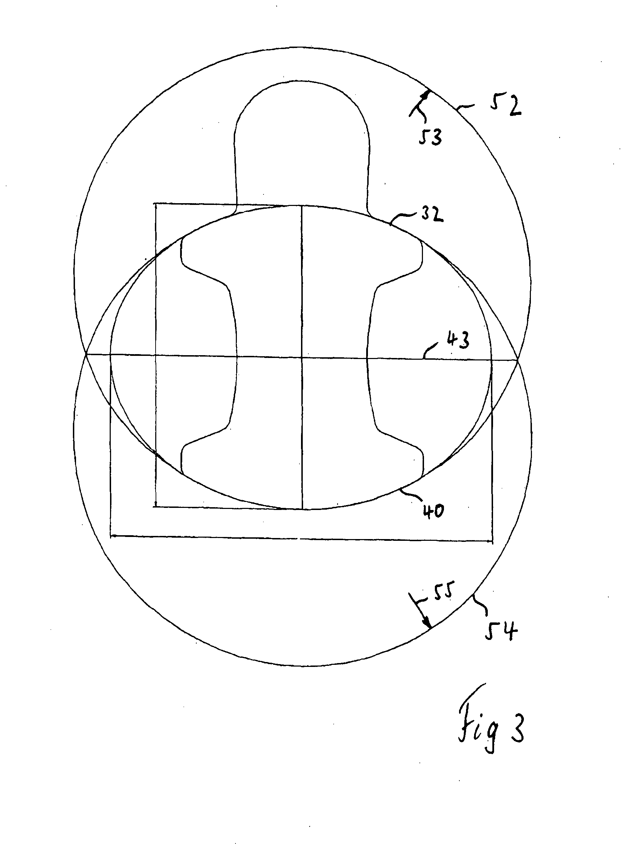

Die vorderseitigen Flanken 32 des vorderen Flügelpaars 12 und die rückseitigen Flanken 40 des hinteren Flügelpaars 14 sind einander entsprechend konturiert. Wenn man die Binde um eine in Querrichtung verlaufende Achse 43 auf sich selbst faltet, so koinzidiert der Verlauf der vorderseitigen Flanken 32 des vorderen Flügelpaars 12 und der rückseitigen Flanken 40 des hinteren Flügelpaars 14. Außerdem folgen im beispielhaft dargestellten Fall die vorderseitigen Flanken 32 des vorderen Flügelpaars 12 und die rückseitigen Flanken 40 des hinteren Flügelpaars 14 ungefähr einer ovalen Form oder einer ungefähren Ellipsenform, deren größere Hauptachse mit der vorerwähnten Achse 43 übereinstimmt. Dieser Verlauf der vorderseitigen Flanken 32 des vorderen Flügelpaars 12 und der rückseitigen Flanken 40 des hinteren Flügelpaars 14 ist in Figur 3 schematisch dargestellt. Die Flanken 32 bzw. 40 verlaufen gekrümmt, deren bogenförmige Krümmung lässt sich auch ungefähr durch Kreise 52 bzw. 54 mit einem Radius 53 bzw. 55 beschreiben, der beispielhaft etwa 150 - 155 mm betragen kann.

Die Aufsichtsfläche ist am Beispiel des rechten Flügels 10 des hinteren Flügelpaars 14 in Figur 1 durch Schraffierung dargestellt. Die Aufsichtsfläche des Flügels 10 beträgt im beispielhaft dargestellten Fall 13 - 14 cm². Die Aufsichtsfläche eines Flügels 8 des vorderen Flügelpaars 12 beträgt ebenso 13 - 14 cm².

Die jeweiligen Übergänge zwischen den Längsrändern 24 des Bindenhauptteils 4 zu der vorderen Flanke 32 und weiter zu dem kürzeren mittleren Flankenabschnitt 36 und weiter zu der rückseitigen Flanke und weiter zu den Längsrändern 24 des Bindenhauptteils 4 und entsprechend bei den Flügeln 10 des hinteren Flügelpaars 14 sind verrundet ausgebildet, wie beispielhaft in Figur 1 dargestellt. Der Krümmungsradius 44 liegt jeweils zwischen 5 und 12 mm.

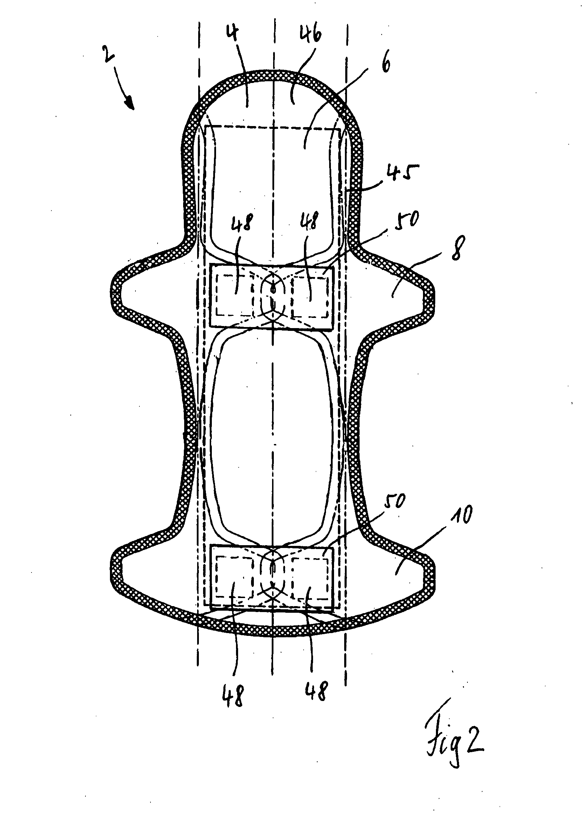

Figur 2 zeigt die Damen- oder Inkontinenzbinde 2 nach Figur 1 und deutet zusätzlich einen herstellerseitig vorgesehenen Einfaltungszustand an, in dem die Flügel 8, 10 um eine in Längsrichtung 16 verlaufende Faltlinie 45 auf eine körperzugewandte Oberseite 46 des Bindenhauptteils 4 eingeschlagen sind. In diesem eingeschlagenen Zustand überlappen die in Querrichtung 18 freien äußeren Enden der Flügel 8 bzw. 10 einander geringfügig im Bereich der Längsmittelachse der Binde. Man erkennt ferner Haftzonen 48, die insbesondere von Klebestreifen oder partiellen Haftkleberbeschichtungen, aber grundsätzlich auch von mechanisch wirkenden Verschlusskomponenten gebildet sein können. Diese Haftzone 48 weist eine Fläche von 20 - 40% der Aufsichtsfläche eines jeweiligen Flügels auf. Diese Haftzonen 48 sind je Flügelpaar 12, 14 von einem einzigen ablösbaren flächenhaften Schutzelement 50, insbesondere in Form eines mit Antihaftmittel beschichteten Papiers überfangen, welches die Flügel 8, 10 in der gefalteten Konfiguration fixiert.

Man erkennt ferner, dass die herstellerseitige Faltlinie 45 geringfügig in Querrichtung 18 außerhalb des Absorptionskörpers 6 der Binde 2 verläuft. Diese Faltlinie wird beim späteren Gebrauch der Damen- oder Inkontinenzbinde 2 typischerweise nicht mit der sich einstellenden Faltung der Flügel 8, 10 um den Steg eines Unterbekleidungsstücks koinzidieren. Je nach Konfektion des Stegbereichs des Unterbekleidungsstücks werden nach dem Lösen der Schutzelemente 50 auch die Flügel 10 des hinteren Flügelpaars 14 um den Steg des Unterbekleidungsstücks gefaltet, oder sie werden von innen gegen die Innenseite des Unterbekleidungsstücks im Gesäßbereich angelegt und vorzugsweise haftend fixiert.

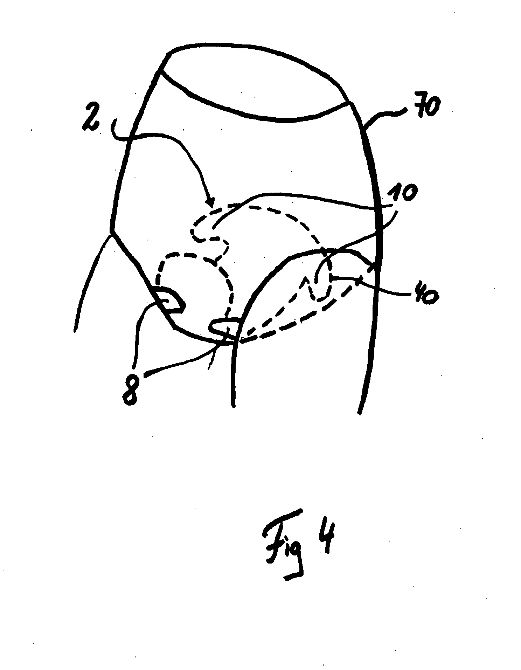

Figur 4 zeigt schematisch die in die Innenseite des Unterbekleidungsstücks 70 eingebrachte Binde 2 im Gebrauchszustand, mit Flügeln 8 des vorderen Flügelpaars, welche um den Steg des Unterbekleidungsstücks herum auf die Außenseite des Unterbekleidungsstücks gefaltet sind und mit Flügeln 10 des hinteren Flügelpaars, welche gegen die Innenseite des Unterbekleidungsstückes anliegen. Die geringe Erstreckung der hinteren Flügel 10 in Längsrichtung der Binde ermöglicht eine größere Flexibilität der hinteren Flügel innerhalb des Unterbekleidungsstückes. Die Krümmung der rückseitigen Flanke 40 des hinteren Flügels 10 unterstützt die ergonomische Passform der Binde 2.

## Patentansprüche

1. Damen- oder Inkontinenzbinde (2) mit um den Steg eines Unterbekleidungsstücks herum faltbaren Flügeln (8, 10), wobei die Binde eine Längsrichtung (16) und eine Querrichtung (18) sowie einen in der Längsrichtung (16) vorderen Endbereich (20), der wenigstens ein Viertel der Gesamtlängserstreckung der Binde erfasst, und einen in der Längsrichtung (16) hinteren Endbereich (22), der höchstens ein Viertel der Gesamtlängserstreckung der Binde erfasst, aufweist, mit einem mit seiner längeren Abmessung in der Längsrichtung (16) erstreckten und einen Absorptionskörper (6) für Körperflüssigkeiten aufnehmenden Bindenhauptteil (4) und mit zwei Flügeln (8, 10) auf jeder Längsseite des Bindenhauptteils (4), die ausgehend von dem Bindenhauptteil (4) in Querrichtung (18) vorstehen, so dass sie ein vorderes und ein hinteres Flügelpaar (12, 14) bilden, wobei das vordere Flügelpaar (12) in Längsrichtung (16) außerhalb des vorderen Endbereichs (20) angeordnet ist, so dass der vordere Endbereich (20) nicht von Flügeln flankiert ist, wobei die Flügel (8, 10) des vorderen und des hinteren Flügelpaars (12, 14) jeweils von einer vorderseitigen und von einer rückseitigen Flanke (32, 34; 38, 40) begrenzt werden, **dadurch gekennzeichnet , dass** wenigstens 90 % der Aufsichtsfläche der Flügel (10) des hinteren Flügelpaars (14) innerhalb des hinteren Endbereichs (22) angeordnet ist.

2. Binde nach Anspruch 1, **dadurch gekennzeichnet, dass** der hintere Endbereich (22) höchstens ein Fünftel, insbesondere höchstens ein Sechstel der Gesamtlängserstreckung der Binde erfasst.

3. Binde nach Anspruch 1 oder 2, **dadurch gekennzeichnet, dass** die Flügel (8) des vorderen Flügelpaars (12) mit wenigstens 90 % ihrer Aufsichtsfläche innerhalb eines Längsabschnittsbereichs (26) der Binde angeordnet sind, der höchstens ein Viertel ihrer Gesamtlängserstreckung erfasst.

4. Binde nach Anspruch 3, **dadurch gekennzeichnet, dass** dieser Längsabschnittsbereich (26) an den vorderen Endbereich (20) anschließt, wobei der vordere Endbereich (20) in Längsrichtung (16) höchstens ein Drittel (1/3), insbesondere höchstens zwei Siebtel (2/7) der Gesamtlängserstreckung der Binde erfasst.

5. Binde nach einem oder mehreren der vorstehenden Ansprüche, **dadurch gekennzeichnet, dass** die Erstreckung der Flügel (8, 10) des vorderen und/oder hinteren Flügelpaars (12, 14) in Querrichtung (18) über die schmalste Stelle (30) der Binde zwischen dem vorderen und dem hinteren Flügelpaar (12, 14) hinaus 20 - 70 mm, insbesondere 20 - 50 mm, insbesondere 25 - 45 mm beträgt.

6. Binde nach einem oder mehreren der vorstehenden Ansprüche, **dadurch gekennzeichnet, dass** die Aufsichtsfläche jedes Flügels (10) des hinteren Flügelpaars (12) und/oder die Aufsichtsfläche jedes Flügels (8) des vorderen Flügelpaars (10) jeweils einen Anteil von 3 - 7 %, weiter vorzugsweise von 4 - 6 % bezogen auf die Gesamtfläche der Damen - oder Inkontinenzbinde einnimmt.

7. Binde nach einem oder mehreren der vorstehenden Ansprüche, **dadurch gekennzeichnet, dass** die Aufsichtsfläche jedes Flügels (8) des vorderen Flügelpaars (12) jeweils 10 - 17 cm², weiter vorzugsweise 11 - 16 cm², weiter vorzugsweise 12 - 15 cm², weiter vorzugsweise 13 - 14 cm² und/oder die jedes Flügels (10) des hinteren Flügelpaars (14) jeweils 10 - 17 cm², weiter vorzugsweise 11 - 16 cm², weiter vorzugsweise 12 - 15 cm², weiter vorzugsweise 13 - 14 cm² beträgt.

8. Binde nach einem oder mehreren der vorstehenden Ansprüche, **dadurch gekennzeichnet, dass** die vorderseitigen und die rückseitigen Flanken (32, 34; 38, 40) der Flügel (8, 10) des vorderen und/oder hinteren Flügelpaars (12, 14) mit dem überwiegenden Teil ihrer Bahn in einem Winkel (α, β) > 50°, insbesondere > 60°, insbesondere > 65° zur Längsrichtung (16) verlaufen, wobei der Winkel weiter insbesondere 60 - 85°, insbesondere 60 - 75° beträgt.

9. Binde nach Anspruch 8, **dadurch gekennzeichnet, dass** die vorderseitigen Flanken (38') der Flügel (10) des hinteren Flügelpaars (14) in einem Winkel > 80°, insbesondere > 90°, insbesondere 90 - 110°, insbesondere 90 - 100° zur Längsrichtung (16) verlaufen.

10. Binde nach einem oder mehreren der vorstehenden Ansprüche, **dadurch gekennzeichnet, dass** zwischen der vorderseitigen und der rückseitigen Flanke (32, 34; 38, 40) eines jeweiligen Flügels (8, 10) ein gegenüber diesen kürzerer Flankenabschnitt (36, 42) vorgesehen ist, der insbesondere im Wesentlichen in Längsrichtung (16) verläuft und in den die Flanken (32, 34; 38, 40) verrundet übergehen.

11. Binde nach einem oder mehreren der vorstehenden Ansprüche, **dadurch gekennzeichnet, dass** ein Krümmungsradius (44) im Übergang zwischen einem im Wesentlichen in der Längsrichtung (16) erstreckten Längsrand (24) des Bindenhauptteils (4) und der vorderseitigen Flanke (32, 38) und/oder zwischen der vorderseitigen Flanke (32, 38) und dem mittleren kürzeren Flankenabschnitt (36, 42) und/oder zwischen dem mittleren kürzeren Flankenabschnitt (36, 42) und der rückseitigen Flanke (34, 40) und/oder zwischen der rückseitigen Flanke (34, 40) und dem im Wesentlichen in der Längsrichtung erstreckten Längsrand (24) des Bindenhauptteils (4) eines jeweiligen Flügels (8, 10) 5 - 12 mm, insbesondere 5 - 10 mm, insbesondere 6 - 9 mm beträgt.

12. Binde nach einem oder mehreren der vorstehenden Ansprüche, **dadurch gekennzeichnet, dass** die rückseitigen Flanken (34) des vorderen Flügelpaars (12) und die vorderseitigen Flanken (38) des hinteren Flügelpaars (14) mit dem überwiegenden Teil ihrer Bahn gerade verlaufen.

13. Binde nach einem oder mehreren der vorstehenden Ansprüche, **dadurch gekennzeichnet, dass** die vorderseitigen Flanken (32) des vorderen Flügelpaars (12) und die rückseitigen Flanken (40) des hinteren Flügelpaars (14) bogenförmig gekrümmt verlaufen.

14. Binde nach einem oder mehreren der vorstehenden Ansprüche, **dadurch gekennzeichnet, dass** die vorderseitigen Flanken (32) des vorderen Flügelpaars (12) und die rückseitigen Flanken (40) des hinteren Flügelpaars (14) einander entsprechend konturiert sind, so dass sie bei Faltung der Binde um eine in Querrichtung verlaufende Achse (43) in Deckung aufeinander bringbar sind.

15. Binde nach Anspruch 14, **dadurch gekennzeichnet, dass** die vorderseitigen Flanken (32) des vorderen Flügelpaars (12) und die rückseitigen Flanken (40) des hinteren Flügelpaars (14) jeweils einer Kreisbahn folgen oder einer Ellipse oder Ovalform folgen, deren größere Hauptachse mit der in Querrichtung verlaufenden Achse (43) zusammenfällt.

16. Binde nach einem oder mehreren der vorstehenden Ansprüche, **dadurch gekennzeichnet, dass** die rückseitigen Flanken (40) des hinteren Flügelpaars (14) in ergonomischer Fortsetzung ihrer Bahn nach innen aufeinander zustreben und so den hinteren Endbereich (22) des Bindenhauptteils (4) begrenzen.

17. Binde nach einem oder mehreren der vorstehenden Ansprüche, **dadurch gekennzeichnet, dass** die Flügel (8, 10) des vorderen und/oder des hinteren Flügelpaars (12, 14) herstellerseitig auf eine körperzugewandte Oberseite (46) der Binde geschlagen sind und einander dabei insbesondere überlappen und dass eine dann nach oben gewandte Haftbeschichtung (48) bei den jeweiligen Flügeln (8, 10) je Flügelpaar (12, 14) von einem einzigen ablösbaren flächenhaften Schutzelement (50), insbesondere auf Papier- oder Folienbasis, insbesondere mit Antihaftmitteln beschichtet, überfangen ist, welches beide Flügel (8, 10) erfasst.

## Claims

1. Sanitary- or incontinence pad (2) with wings (8, 10) which can be folded about the web of an undergarment, wherein the pad has a longitudinal direction (16) and a transverse direction (18) and an front end region (20) in the longitudinal direction (16) which covers at least one quarter of the overall longitudinal extension of the pad, and a rear end region (22) in the longitudinal direction which covers at most one quarter of the overall longitudinal extension of the pad, with a primary pad part (4) which extends in the longitudinal direction (16) with its longer dimension and accommodates an absorption body (6) for bodily fluids and with two wings (8, 10) on each longitudinal side of the primary pad part (4) which protrude in transverse direction (18) starting from the primary pad part (4) so that they form a front and a rear wing pair (12, 14) wherein the front wing pair is arranged outside of the front end region (20) in longitudinal direction (16) so that the front end region (20) is not flanked by wings, wherein the wings (8, 10) of the front and the rear wing pairs (12, 14) are each delimited by a front and a rear side flank (32, 34; 38, 40), **characterized in that** at least 90 % of the top-view-surface of the wings (10) of the rear wing pair (14) are arranged within the rear end region (22).

2. Pad according to claim 1, **characterized in that** the rear end region (22) covers at most one fifth in particular at most one sixth of the overall longitudinal extension of the pad.

3. Pad according to claim 1 or 2, **characterized in that** the wings (8) of the front wing pair (12) are arranged within a longitudinal section (26) of the pad with at least 90 % of their top-view-surface, which longitudinal section covers at most one quarter of its overall longitudinal extension.

4. Pad according to claim 3, **characterized in that** this longitudinal section (26) adjoins the front ends wherein the front end region in longitudinal direction covers at most one third (1/3) in particular at most two seventh (2/7) of the overall longitudinal extension of the pad.

5. Pad according to one or multiple of the preceding claims, **characterized in that** the extension of the wings (8, 10) of the front and/or rear wing pair (12, 14) in transverse direction (18) past the narrowest point (30) of the pad between the front and the rear wing pair (12, 14) is 20 - 70 mm, in particular 20 - 50 mm, in particular 25 - 45 mm.

6. Pad according to one or more of the preceding claims, **characterized in that** the top-view-surface of each wing (10) of the rear wing pair (12) and/or the top-view-surface of each wing (8) of the front wing pair (10) each occupy a portion of 3 - 7%, further preferably of 4.- 6% relative to the overall surface of the sanitary or incontinence pad.

7. Pad according to one or more of the preceding claims, **characterized in that** the top-view-surface of each wing (8) of the front wing pair (12) each is 10 - 17 cm², further preferably 11 - 16 cm², further preferably 12 - 15 cm², further preferably 13 - 14 cm² and/or the top-view-surface of each wing of the rear wing pair (14) each is each is 10 - 17 cm², further preferably 11 - 16 cm², further preferably 12 - 15 cm², further preferably 13 - 14 cm² .

8. Pad according to on or more of the preceding claims, **characterized in that** the front side and rear side flanks (32, 34; 38, 40) of the wings (8, 10) of the front and/or rear wing pair (12, 14) extend with the predominant portion of their track at an angle (α, β) > 50°, in particular >60°, in particular >65° relative to the longitudinal direction (16), wherein the angle is further in particular 60 - 85°, in particular 60 - 75°.

9. Pad according to one or more of the preceding claims, **characterized in that** the front side flanks (38') of the wings (10) of the rear wing pair (14) extend at an angle of >80°, in particular >90°, in particular 90 - 110°, in particular 90 - 110° relative to the longitudinal direction (16).

10. Pad according to one or more of the preceding claims, **characterized in that** a flank section (36, 42) is provided between the front side and the rear side flank (32, 34; 38, 40) of a respective wing (8, 10) which flank section is shorter than the front and the rear side flank and extends in particular substantially in longitudinal direction (16), and into which the flanks (32, 34; 38, 40) transition in a rounded manner.

11. Pad according to one or more of the preceding claims, **characterized in that** a curvature radius (44) in the transition between a longitudinal border of the primary pad part (4), which extends substantially in the longitudinal direction (16) and the front side flank (32, 38) and/or between the front side flank (32, 38) and the center shorter flanks section (36, 42) and or between the center shorter flank section (36, 42) and the rear side flank (34, 40) and/or between the rear side flank (34, 40) and the longitudinal border (24) of the primary pad part (4), which substantially extends in the longitudinal direction of a respective wing (8, 10) is 5 - 12 mm, in particular 5 - 10 mm, in particular 6 - 9 mm.

12. Pad according to one or more of the preceding claims, **characterized in that** the rear side flanks (34) of the front wing pair (12) and the front side flanks (38) of the rear wing pair (14) extend straight with the predominant portion of their track.

13. Pad according to one or more of the preceding claims, **characterized in that** the front side flanks (32) of the front wing pair (12) and the rear side flanks (40) of the rear wing pair (14) extend curved in the shape of an arch.

14. Pad according to one or more of the preceding claims, **characterized in that** the front side flanks (32) of the front wing pair (12) and the rear side flanks (40) of the rear wing pair (14) are contoured so as to correspond to one another, so that they can be brought to align with one another when folding the pad about an axis which extends in transverse direction.

15. Pad according to claim 14, **characterized in that** the front side flanks (32) of the front wing pair (12) and the rear side flanks (40) of the rear wing pair (14) each follow a circular path or an ellipsis or oval shape, whose greater main axis coincides with the axis (43) which extends in transverse direction.

16. Pad according to one or more of the preceding claims, **characterized in that** the rear side flanks (40) of the rear wing pair (14) extend inward toward one another in extension of their ergonomic track and thereby delimit the rear end region (22) of the primary pad part (4).

17. Pad according to on or more of the preceding claims, **characterized in that** the wings (8, 10) of the front and/or the rear wing pair (12, 14) are folded onto a body facing topside (46) of the pad by the manufacturer and in particular overlap one another and **in that** an adhesive coating (48) which is then facing upward at the respective wings (8, 10) per wing pair (12, 14) is covered by a single detachable flat protective element (50) in particular on paper or foil basis, which is in particular coated with anti stick means, and which covers both wings (8, 10).

## Revendications

1. Serviette hygiénique ou de protection contre l'incontinence (2), ayant des ailettes (8, 10) aptes à être repliées autour de l'entrejambe d'un sous-vêtement, ladite serviette présentant une direction longitudinale (16) et une direction transversale (18) ainsi qu'une zone terminale (20) avant dans ladite direction longitudinale (16), qui comprend au moins un quart de l'extension longitudinale totale de la serviette, et une zone terminale (22) arrière dans ladite direction longitudinale (16), qui comprend tout au plus un quart de l'extension longitudinale totale de la serviette, comprenant une partie principale de serviette (4) qui, avec sa dimension plus longue, s'étend dans la direction longitudinale (16) et qui reçoit un corps d'absorption (6) pour fluides corporels, ainsi que deux ailettes (8, 10) de chaque grand côté de ladite partie principale de serviette (4), qui font saillie dans la direction transversale (18) à partir de la partie principale de serviette (4) de sorte qu'elles forment des paires d'ailettes avant et arrière (12, 14), la paire d'ailettes avant (12) étant disposée hors de la zone terminale avant (20) dans la direction longitudinale (16) de sorte que ladite zone terminale avant (20) n'est pas flanquée d'ailettes, les ailettes (8, 10) des paires d'ailettes avant et arrière (12, 14) étant délimitées chacune d'un flanc frontal et d'un flanc arrière (32, 34; 38, 40), **caractérisée par le fait qu'**au moins 90 % de la surface de vue en plan des ailettes (10) de la paire d'ailettes arrière (14) sont disposés à l'intérieur de ladite zone terminale arrière (22).

2. Serviette selon la revendication 1, **caractérisée par le fait que** ladite zone terminale arrière (22) comprend tout au plus un cinquième, en particulier tout au plus un sixième de l'extension longitudinale totale de la serviette.

3. Serviette selon la revendication 1 ou 2, **caractérisée par le fait qu'**au moins 90 % de la surface de vue en plan des ailettes (8) de la paire d'ailettes avant (12) sont disposés à l'intérieur d'une zone de portion longitudinale (26) de la serviette, qui comprend tout au plus un quart de l'extension longitudinale totale de celle-ci.

4. Serviette selon la revendication 3, **caractérisée par le fait que** cette zone de portion longitudinale (26) est contiguë à ladite zone terminale avant (20), la zone terminale avant (20) comprenant, dans la direction longitudinale (16), tout au plus un tiers (1/3), en particulier tout au plus deux septièmes (2/7) de l'extension longitudinale totale de la serviette.

5. Serviette selon l'une ou plusieurs des revendications précédentes, **caractérisée par le fait que** l'extension des ailettes (8, 10) des paires d'ailettes avant et/ou arrière (12, 14) dans la direction transversale (18), au-delà de l'endroit (30) le plus étroit de la serviette entre les paires d'ailettes avant et arrière (12, 14), est comprise entre 20 et 70 mm, en particulier entre 20 et 50 mm, en particulier entre 25 et 45 mm.

6. Serviette selon l'une ou plusieurs des revendications précédentes, **caractérisée par le fait que** la surface de vue en plan de chaque ailette (10) de la paire d'ailette arrière (14) et/ou la surface de vue en plan de chaque ailette (8) de la paire d'ailettes avant (12) occupe chacune une part comprise entre 3 et 7 %, de préférence encore entre 4 et 6 % par rapport à la surface totale de la serviette hygiénique ou de protection contre l'incontinence.

7. Serviette selon l'une ou plusieurs des revendications précédentes, **caractérisée par le fait que** la surface de vue en plan de chaque ailette (8) de la paire d'ailettes avant (12) fait chacune entre 10 et 17 cm², de préférence encore entre 11 et 16 cm², de préférence encore entre 12 et 15 cm², de préférence encore entre 13 et 14 cm², et/ou celle de chaque ailette (10) de la paire d'ailettes arrière (14) fait chacune entre 10 et 17 cm², de préférence encore entre 11 et 16 cm², de préférence encore entre 12 et 15 cm², de préférence encore entre 13 et 14 cm².

8. Serviette selon l'une ou plusieurs des revendications précédentes, **caractérisée par le fait que** la majeure partie de la voie des flancs frontaux et des flancs arrière (32, 34; 38, 40) des ailettes (8, 10) des paires d'ailettes avant et/ou arrière (12, 14) s'étend à un angle (α, β) > 50°, en particulier > 60°, en particulier > 65° par rapport à la direction longitudinale (16), ledit angle étant, en particulier encore, compris entre 60 et 85°, en particulier entre 60 et 75°.

9. Serviette selon la revendication 8, **caractérisée par le fait que** les flancs frontaux (38') des ailettes (10) de la paire d'ailettes arrière (14) s'étendent à un angle > 80°, en particulier > 90°, en particulier entre 90 et 110°, en particulier entre 90 et 100° par rapport à la direction longitudinale (16).

10. Serviette selon l'une ou plusieurs des revendications précédentes, **caractérisée par le fait qu'**entre le flanc frontal et le flanc arrière (32, 34; 38, 40) d'une ailette (8, 10) respective est prévue une portion de flanc (36, 42) qui est plus courte par rapport à ceux-ci et s'étend, en particulier, pour l'essentiel dans la direction longitudinale (16) et auquel lesdits flancs (32, 34; 38, 40) sont contigus tout en étant arrondis.

11. Serviette selon l'une ou plusieurs des revendications précédentes, **caractérisée par le fait qu'**un rayon de courbure (44) dans la transition entre un bord longitudinal (24) de la partie principale de serviette (4), qui s'étend pour l'essentiel dans la direction longitudinale (16), et le flanc frontal (32, 38) et/ou entre le flanc frontal (32, 38) et ladite portion de flanc (36, 42) centrale plus courte et/ou entre ladite portion de flanc (36, 42) centrale plus courte et le flanc arrière (34, 40) et/ou entre le flanc arrière (34, 40) et le bord longitudinal (24) de la partie principale de serviette (4) d'une ailette (8, 10) respective, qui s'étend pour l'essentiel dans la direction longitudinale, est compris entre 5 et 12 mm, en particulier entre 5 et 10 mm, en particulier entre 6 et 9 mm.

12. Serviette selon l'une ou plusieurs des revendications précédentes, **caractérisée par le fait que** la majeure partie de la voie des flancs arrière (34) de la paire d'ailettes avant (12) ainsi que des flancs frontaux (38) de la paire d'ailette arrière (14) s'étend de manière droite.

13. Serviette selon l'une ou plusieurs des revendications précédentes, **caractérisée par le fait que** les flancs frontaux (32) de la paire d'ailettes avant (12) et les flancs arrière (40) de la paire d'ailette arrière (14) s'étendent de manière courbée en arc.

14. Serviette selon l'une ou plusieurs des revendications précédentes, **caractérisée par le fait que** les flancs frontaux (32) de la paire d'ailettes avant (12) et les flancs arrière (40) de la paire d'ailette arrière (14) présentent des contours qui correspondent entre eux de sorte que, lorsque la serviette est pliée sur un axe (43) s'étendant dans la direction transversale, ils puissent être mis en coïncidence l'un sur l'autre.

15. Serviette selon la revendication 14, **caractérisée par** le fait les flancs frontaux (32) de la paire d'ailettes avant (12) et les flancs arrière (40) de la paire d'ailettes arrière (14) suivent chacun une trajectoire circulaire ou suivent une ellipse ou forme ovale dont l'axe principal plus grand coïncide avec l'axe (43) s'étendant dans la direction transversale.

16. Serviette selon l'une ou plusieurs des revendications précédentes, **caractérisée par le fait que** les flancs arrière (40) de la paire d'ailettes arrière (14), en prolongement ergonomique de leur voie, s'étendent vers l'intérieur l'un en direction de l'autre et délimitent ainsi la zone terminale arrière (22) de la partie principale de serviette (4).

17. Serviette selon l'une ou plusieurs des revendications précédentes, **caractérisée par le fait que** les ailettes (8, 10) des paires d'ailettes avant et/ou arrière (12, 14) sont rabattues, de la part du fabricant, sur une face supérieure (46) de la serviette qui montre vers le corps et, se faisant, en particulier se chevauchent les unes les autres, et qu'un revêtement adhésif (48) montrant alors vers le haut, dans les ailettes (8, 10) respectives par paire d'ailettes (12, 14), est recouvert d'un seul élément protecteur en nappe (50) détachable, en particulier à base de papier ou de feuille, en particulier revêtu d'agents antiadhésifs, qui s'étend sur les deux ailettes (8, 10).
